# EUROPÄISCHE PATENTSCHRIFT

(11) **EP 1 755 630 B1**
(45) Veröffentlichungstag und Bekanntmachung des Hinweises auf die Patenterteilung: **24.10.2007**
(21) Anmeldenummer: 05731107.8
(22) Anmeldetag: 07.04.2005
(51) Int. Cl.: A61K 36/185, A61K 36/09, A61P 17/00, A61P 17/06

(54) **PHARMAZEUTISCHE ZUSAMMENSETZUNGEN AUS BARTFLECHTEN (USNEA BARBATA) UND JOHANNISKRAUT (HYPERICUM PERFORATUM) SOWIE IHRE VERWENDUNG**
PHARMACEUTICAL COMPOSITIONS FROM BEARD LICHEN (USNEA BARBATA) AND ST. JOHN'S WORT (HYPERICUM PERFORATUM) AND THEIR USE
COMPOSITIONS PHARMACEUTIQUES CONTENANT DE L'USNEE BARBATA ET ET DE L'HYPERICUM PERFORATUM, ET LEUR UTILISATION

(30) Priorität: 19.04.2004 EP 04009213
(43) Veröffentlichungstag der Anmeldung: 28.02.2007
(73) Patentinhaber: Universitätsklinikum Freiburg, 79106 Freiburg (DE)
(72) Erfinder: SCHEMPP, Christoph, 79103 Freiburg (DE); JOCHER, Andrea, 79106 Freiburg (DE); ENGEL, Kathrin, 79224 Umkirch (DE); HUYKE, Constance, 79117 Freiburg (DE)
(74) Vertreter: Keller, Günter
(86) Internationale Anmeldenummer: PCT/EP2005/003657
(87) Internationale Veröffentlichungsnummer: WO 2005/099728

(56) Entgegenhaltungen:
- WO-A-02/094300
- DE-A1- 10 131 641
- COSMETICS AND TOILETRIES MANUFACTURE, 2002, Seiten 43-48, XP009048615 Aston Publishing Group
- PATENT ABSTRACTS OF JAPAN Bd. 1995, Nr. 03, 28. April 1995 (1995-04-28) & JP 06 345625 A (KAO CORP), 20. Dezember 1994 (1994-12-20)
- MADAMOMBE I T ET AL: "Evaluation of antimicrobial activity of extracts from South African Usnea barbata." Mai 2003 (2003-05), PHARMACEUTICAL BIOLOGY, VOL. 41, NR. 3, PAGE(S) 199-202 , XP009048617 ISSN: 1388-0209 Seite 201

## Beschreibung

Die vorliegende Erfindung betrifft pharmazeutische Zusammensetzungen mit Bestandteilen aus Usnea barbata, die insbesondere bei solchen Hauterkrankungen eingesetzt werden können, bei denen es durch Einwirken von Sonnenstrahlen und Sauerstoffradikalen zu Entzündungen und zu einer beschleunigten Hautalterung kommt. Die pharmazeutischen Zusammensetzungen können auch bei solchen Hauterkrankungen eingesetzt werden, bei denen verschiedene Hefepilze und Bakterien zum Entstehen dieser Krankheiten beitragen.

Zu den Hauterkrankungen des seborrhoischen Formenkreises gehören Akne, Rosacea und das seborrhoische Ekzem. Verschiedene Bakterien, insbesondere Propionibacterium acnes oder Corynebacterien, und Hefepilze, insbesondere Pityrosporon, tragen zum Entstehen dieser Hautkrankheiten bei. Ausserdem sind die Krankheiten des seborrhoischen Formenkreises durch Entzündungen der Haarfollikel und des umgebenden Gewebes gekennzeichnet. Diese Hautkrankheiten beeinträchtigen die Lebensqualität der betroffenen Personen erheblich. Leichte bis mittelschwere Formen werden normalerweise mit verschiedenen Lokaltherapeutika behandelt. Zu den üblichen Behandlungsmitteln gehören Peelings (z.B. Benzoylperoxid, Glykolsäure), Antibiotika (z.B. Erythromycin, Clindamycin, Tetrazyklin) und Vitamin A-Säure-Derivate.

Diese üblichen Wirkstoffe sind aus verschiedenen Gründen problematisch: Die lokale Anwendung der Antibiotika ist zwar wirksam, jedoch können Resistenzen entstehen. Diese wiederum sind problematisch, da die Antibiotika auch bei anderen schweren Infektionen eingesetzt werden. Benzoylperoxid führt zu Hautreizungen und bleicht außerdem Wäsche. Vitamin A Säure führt ebenfalls zu ausgeprägten Hautreizungen. Die eingesetzten Substanzen haben keine nennenswerte entzündungshemmende Wirkung.

Hauterkrankungen des seborrhoischen Formenkreises (Akne) sind häufig sehr problematisch. Da die Hautirritationen vor allem im Gesicht auftreten, kann dies zu schweren psychischen Störungen führen. Es besteht daher ein Bedürfnis nach wirksamen pharmazeutischen Zusammensetzungen, die einerseits die unerwünschten Symptome wirksam und schnell beseitigen und andererseits möglichst geringfügige Nebenwirkungen haben.

Aus dem Stand der Technik sind verschiedene Präparate bekannt, die Pflanzenbestandteile als Wirkstoffe enthalten.

WO02/094300, "Herbal Compositions for the Treatment of mucosal Lesions" beschreibt therapeutische Zusammensetzungen, die nützlich sind bei der Bekämpfung von entzündlichen Schleimhauterkrankungen viralen Ursprungs. Die Zusammensetzungen enthalten Extrakte der Pflanzenarten Echinacea purpurea und Sambucus nigra sowie Extrakte mindestens einer weiteren Pflanze, ausgewählt aus einer Gruppe, die besteht aus Hypericum perforatum, Commiphora molmol und Centella asiatica. Diese Basiskomposition kann weitere Pflanzenextrakte enthalten, bestehend aus der Gruppe von Uncaria tomentosa, Thymus vulgaris, Matricaria recutita, Salix alba, Calendula officinalis, Usnea barbata, Ligusticum porterii, Gaultheria procumbens, Camellia sinensis, Vaccinium myrtillus, Melissa officinalis, Allium sativum und Crameria triandra. Aus den möglichen Kombinationen von 19 verschiedenen Pflanzen ist nicht ersichtlich, welche Kombination eine besondere Wirkung aufweist. Desweiteren wird in WO02/094300 an keiner Stelle der Formenkreis seborrhoischer Erkrankungen und speziell der Akne genannt. Es wird vielmehr von viral bedingten entzündlichen Schleimhauterkrankungen, von anderen entzündlichen Schleimhauterkrankungen und von Insektenstich-Reaktionen gesprochen.

In der JP 52468876 wird beschrieben, dass Flechtenextrakte antibakterielle Effekte gegen grampositive Bakterien aufweisen können.

WO 02/051427 beschreibt stabile Extrakte aus Hypericum perforatum sowie pharmazeutische Zusammensetzungen, die diesen Extrakt enthalten, und topische Arzneimittel zur Behandlung von Akne, atopischer Dermatitis, Psoriasis usw.

DE - 101 31 641 verwendet einen Extrakt aus Hypericum perforatum L., darin enthalten eine Kombination aus Hyperforin, Hypericin und Flavonen zur antiphlogistischen, topischen Anwendung. Eine Kombination dieser Wirkstoffe mit Usninsäure ist nicht beschrieben.

JP 06 345 625 offenbart die Kombination eines Hypericum-Extraktes mit einem Anti-Seborrhoe-Wirkstoff im Einsatz zur Haarpflege.

Madamombe et al., Pharmaceutical Biology (2003), S. 199-202 offenbart die bekannten, antimikrobiellen Eigenschaften von Usnea-Arten gegen gram-positive Bakterien. Hierbei wird ein Totalextrakt mit Aceton, Methanol oder Wasser eingesetzt. Hyperforin wird nicht erwähnt.

Quirin, Cosmetics and Toileteries Manufacture, 2002, S. 43-48 offenbart CO₂-Extrakte aus Usnea-Arten und Hypericum perforatum. Eine Kombination aus diesen beiden Extrakten wird aber nirgends erwähnt oder nahegelegt.

EP 1131063 beschreibt die Verwendung von Hyperforin aus Hypericum perforatum als antibakteriellen Inhaltsstoff, der sich zur Behandlung von Hautkrankheiten eignet.

Erfindungsgemäß wurde gefunden, dass die Kombination von Extrakten aus Usnea barbata und Hypericum perforatum zu einer unerwarteten Potenzierung der antiinflammatorischen Wirkung der Einzelkomponenten beiträgt. Die Kombination der Extrakte führt ausserdem zu einer vollständigen Hemmung der UV-induzierten Aktivierung der Collagenase. Besonders überraschend ist, dass die Kombination der Extrakte aus Usnea barbata und Hypericum perforatum zu einer unerwarteten Stabilisierung des für die Wirkung wichtigen Hyperforins führt. Auf diese Weise können die entzündungshemmenden und antimikrobiellen Wirkungen der beiden Einzelkomponenten vereinigt und potenziert werden.

Bestrahlung mit Ultraviolett-B (UVB)-Strahlen führt zu einer Rötung und Entzündung der Haut, die in stärkerer Ausprägung als Sonnenbrand (Dermatitis solaris) bezeichnet wird. Ein wichtiger Faktor für die Entstehung von Sonnenbrand ist die Produktion von Prostaglandin E₂ (PGE₂) durch Keratinozyten. Eine wichtige Rolle bei der UV-induzierten Entzündung spielen Sauerstoffradikale. Diese führen über eine Aktivierung des epidermalen Wachstums-Faktor (EGF)-Rezeptors zu einer Aufreguation der Cyclooxygenase-2 (COX-2). COX-2 katalysiert die Bildung von PGE₂ (Ashida et al., Experimental Dermatology 2003:12:445-452).

Erfindungsgemäß wird eine wirksame Formulierung offenbart, die die UV-induzierte PGE₂-Synthese hemmen kann. Es wurde überraschend gefunden, dass ein Extrakt aus Usnea barbata mit einem definierten Gehalt an Usninsäure die UV-induzierte Synthese von PGE₂ in Keratinozyten hemmt.

UV-Bestrahlung der Haut führt auch zu einer beschleunigten Hautalterung. Durch die Bildung von Sauerstoffradikalen und proinflammatorischen Zytokinen wird im Bindegewebe der Haut in den Fibroblasten die Aktivierung des Enzyms Matrix-Metalloproteinase-1 (MMP-1) induziert (Scharfetter et al., Archives of Dermatological Research 1991: 283: 506-511). MMP-1 wird auch als Collagenase bezeichnet. Die Collagenase führt zu einem beschleunigten Abbau von Kollagen-1, das ein wichtiger Bestandteil des Bindegewebes der Haut ist. Durch die Aktivierung der Collagenase kommt es zu einer vorzeitigen, beschleunigten Hautalterung.

Erfindungsgemäß wird eine wirksame Formulierung bereitgestellt, die die UV-induzierte MMP-1 Collagenase-Aktivierung hemmen kann. Es wurde überraschend gefunden, dass die UV-induzierte Aufregulation der Collagenase in Fibroblasten durch einen Extrakt aus Usnea barbata gehemmt werden kann. Diese Wirkung konnte durch die Kombination des Extrakts aus Usnea barbata mit einem Extrakt aus Hypericum perforatum weiter potenziert werden.

Gegenstand der vorliegenden Erfindung sind pharmazeutische Zusammensetzungen, die dadurch gekennzeichnet sind, dass sie 0,001 - 20 Gew.-% und bevorzugt 0,01 - 10 Gew.-% eines CO₂-Extrakte aus Usnea barbata und 0,01 - 80 Gew.-%, bevorzugt 0,01 - 20 Gew.-% eines CO₂-Extraktes aus Johanniskraut (Hypericum perforatum) enthalten. Die Gewichtsprozentangaben beziehen sich auf die fertige pharmazeutische Zusammensetzung.

Erfindungsgemäß wird ein CO₂-Extrak aus Usnea barbata (Bartflechte) eingesetzt. Hauptbestandteil der wirksamen Komponenten des Extrakts ist die Usninsäure, die in zwei enantiomeren Formen, nämlich der (+)-9bR-Usninsäure und der (-)-9bS-Usninsäure vorkommt. Man geht davon aus, dass vor allem die (+)-Usninsäure wegen ihrer bioziden Wirkung in der Pharmazie und Kosmetik bevorzugt verwendet wird. Im vorliegenden Fall wird bevorzugt das Racemat oder die (+)-9bR-Usninsäure eingesetzt.

Verfahren zur Isolierung von Usninsäure aus Flechten (Usnea barbata) sind im Stand der Technik bekannt (beispielsweise DE 32 13 095). Bei diesen Extraktionsverfahren wird mit organischen Lösungsmitteln oder Alkohol gearbeitet.

Ein Nachteil dieser Methoden ist, dass keine Abreicherung von Schadstoffen durch die Extraktion erfolgt. Dies ist wichtig, da Bartflechten Schadstoffe aus der Luft und aus Niederschlägen anreichern können (beispielsweise Schwermetalle). Bevorzugt eingesetzt werden im Rahmen der vorliegenden Erfindung deshalb solche Extrakte aus Usnea barbata, die durch eine Hochdruckextraktion mit CO₂ hergestellt wurden. Ein Vorteil dieser Extraktionsmethode ist, dass keine Schwermetalle und Lösungsmittelrückstände zurückbleiben. Durch die CO₂-Extraktion kann die Usninsäure zu einer praktisch reinen Form aufgereinigt werden.

Eingesetzt werden können Extrakte aus Usnea barbata, die durch eine Hochdruckextraktion mit CO₂ hergestellt wurden. Vorteil dieser Extraktionsmethode ist, dass keine Lösungsmittelrückstände zurückbleiben. Nach der CO₂-Extraktion kann die Usninsäure zu einer praktisch reinen Form aufgereinigt werden. Die Extrakte werden mit Pflanzenöl und Emulgatoren formuliert und sind käuflich erhältlich (beispielsweise Flavex^{®} Naturextrakte GmbH). Durch Zugabe von pharmazeutisch annehmbaren Zusatzmitteln kann der gewünschte Gehalt an Wirkstoff eingestellt werden.

Die eingesetzten Extrakte aus Flechten enthalten 2-7, bevorzugt 3-5 und besonders bevorzugt etwa 4 Gew.-% Usninsäure. In dem CO₂-Extrakt liegen auch Derivate der Usninsäure als weitere Flechtensäure in einer Menge von bis zu 0,5 Gew.-%, bevorzugt bis zu 0,2 Gew.-% und besonders bevorzugt bis zu 0,1 Gew.-% vor.

Der erfindungsgemäß bevorzugt eingesetzte Extrakt aus Usnea barbata wird nach einem speziellen Verfahren mit Quellkohlensäure unter Hochdruck hergestellt. Die Droge (Pflanzenmaterial) wird dabei nach vorheriger Konditionierung mit überkritischem CO₂ extrahiert. Der so erhaltene Rohextrakt enthält etwa 60 % Usninsäure. In einem zweiten Reinigungsschritt werden die unwirksamen oder unerwünschten lipophilen Begleitstoffe wie flüchtige Öle, Fettsäuren, Kohlenwasserstoffe, Chlorophylle und andere Flechtensäuren von der Usninsäure weitgehend abgetrennt.

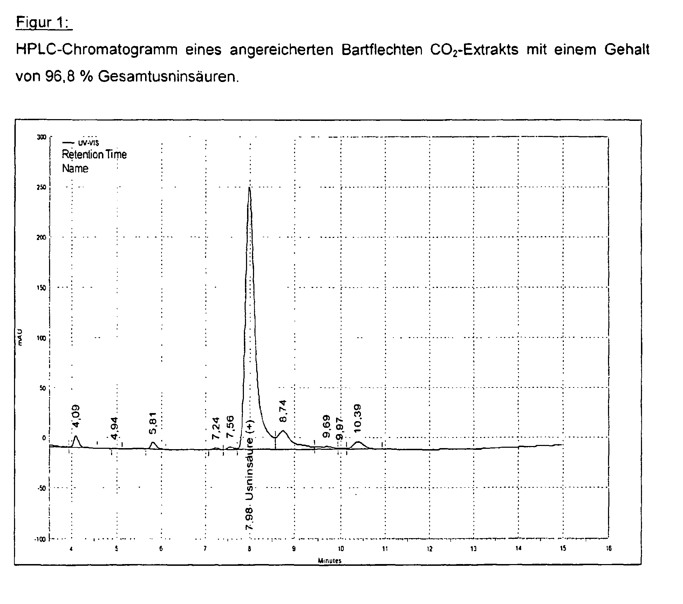

Dadurch gelangt man zu dem erfindungsgemäß bevorzugten CO₂-Extrakt mit etwa 92 +/-5 % Gehalt an Usninsäuren, der als helles gelbliches bis leicht grünliches Pulver anfällt, während die aus dem Rohextrakt separierten Komponenten ein dunkelgrünes viskoses Öl bilden, das verworfen wird. Damit ist sicher gestellt, dass eventuell vorhandene, potentiell allergene Flechtensäuren vom Depsidontyp oder aliphatische Flechtensäuren, die durch das Vorhandensein von ein oder zwei Carboxygruppen charakterisiert sind, von der natürlichen Usninsäure, einem Dibenzofuranderivat ohne Carboxygruppe weitgehend abgetrennt werden. Ein HPLC Chromatogramm des gereinigten Bartflechten CO₂-Extrakts mit 96,8 % Gesamtusninsäuren zeigt Fig. 1, wobei die Gesamtusninsäuren aus 88,3 % (+/-)-Usninsäure und 8,5 % eines Usninsäurederivates (Iso-Usninsäure) bestehen.

Für die Verwendung in pharmazeutischen Zubereitungen kann es erforderlich werden, die Wirkstoffe in niedrigen Konzentrationen anzuwenden. In diesem Fall werden die Extrakte mit geeigneten Verdünnungsmitteln wie beispielsweise Triglyceriden und Emulgatoren auf niedrige Wirkstoffkonzentrationen (beispielsweise 4 % Usninsäure, 10 % Hyperforin) verdünnt.

Obwohl Allergien gegen Flechtensäuren beschrieben sind, gilt die sensibilisierende Potenz der Flechten als gering und wird durch andere Flechtensäuren als Usninsäure bedingt. Daher werden bevorzugt gereinigte CO₂-Extrakte eingesetzt, die einen Gesamtusninsäuregehalt von mindestens 85 % aufweisen. Bevorzugt weisen diese CO₂-Extrakte höchstens 0,1 Gew.-% und besonders bevorzugt weniger als 0,1 Gew.-% an anderen Flechtensäuren auf, die für Allergien verantwortlich sein können.

In der Literatur wird beschrieben, dass Usnea-Arten und Usninsäure ein breites antibakterielles Spektrum aufweisen, und dass sie sich deshalb zur Behandlung von Oberflächeninfektionen und Hautulzera eignen. Die Übersicht von Cocchietto M et al. (2002, Naturwissenschaften 89: 137-146) fasst die Erkenntnisse zu den antibakteriellen Wirkungen von Usninsäure zusammen.

Der andere, wesentliche Bestandteil der erfindungsgemäßen pharmazeutischen Zusammensetzungen ist ein Extrakt aus Johanniskraut (Hypericum perforatum). Das Johanniskraut wird in großem Umfang und seit langem in der Phytomedizin verwendet. Ein wesentlicher Bestandteil des Johanniskrauts ist der Wirkstoff Hyperforin.

Da im Johanniskraut besonders die Wirkungen des Hyperforins für die erfindungsgemässe Verwendung bedeutsam sind, wird ein CO₂-Extrak aus Hypericum perforatum verwendet. CO₂-Extrakte aus Hypericum perforatum enthalten als wirksame Bestandteile praktisch nur Hyperforine aus der Gruppe der Acylphloroglucinole, aber praktisch keine Hypericine aus der Gruppe der Naphthodianthrone. CO₂-Extrakte enthalten ebenso keine Flavonolglycoside, Proanthocyanidine, Chlorophylle und Chlorogensäuren.

Hyperforin ist ein antibakterieller Inhaltsstoff aus dem Johanniskraut (Hypericum perforatum), der sich zur Behandlung entzündlicher Hautkrankheiten eignet (EP 1 131 063). Dieser Inhaltsstoff ist aufgrund seiner ausgeprägten Lipophilie nur in apolaren Lösungsmitteln und Fetten gut löslich. Bei Emulgation in wässrigen Lösungen tritt ein rascher Abbau des Hyperforins auf. Dieses Problem kann umgangen werden, indem verschiedene Salze des Hyperforins hergestellt werden. In dieser Form kann Hyperforin stabil gelagert werden (WO 99/41220). Werden jedoch die Hyperforinsalze in eine wässrige Lösung gebracht, dissoziiert freies Hyperforin und wird ebenfalls rasch abgebaut.

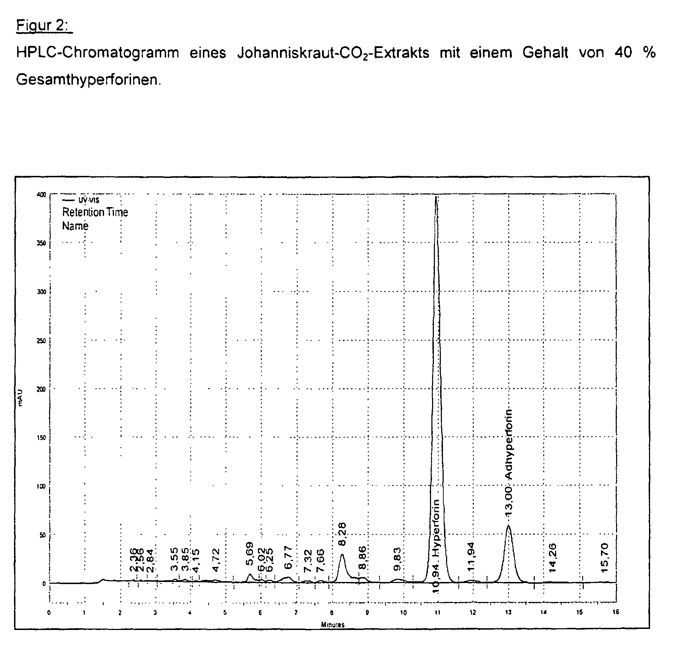

Erfindungsgemäß wird bevorzugt ein CO₂-Extrakt aus Hypericum perforatum eingesetzt. Aus den Zweigspitzen und Blüten von Hypericum perforatum wird durch Hochdruckextraktion mit Quellkohlensäure ein Extrakt hergestellt. Es ist bekannt, dass die blühenden Zweigspitzen in der frühen Vegetationsperiode vorwiegend Hypericine enthalten, während im späteren Blütenstadium die Hyperforine überwiegen. Setzt man einen diesbezüglich optimierten Rohstoff ein, der für die Extraktion schonend aufbereitet und konditioniert ist, ist es möglich, CO₂-Extrakte zu gewinnen, die einen Gesamthyperforingehalt von bis zu 40 % aufweisen. Das Gesamthyperforin besteht normalerweise aus 82-86 % Hyperforin und 14-18 % Adhyperforin. Ein HPLC-Chromatogramm eines hochwertigen CO₂-Extrakts mit 40 % Gesamthyperforin, bestehend aus 34,1 % Hyperforin und 5,9 % Adhyperforin zeigt Fig. 2.

In bevorzugter Ausführungsform enthält der erfindungsgemäß eingesetzte Johanniskrautextrakt kein nachweisbares Hypericin. Bei Anwendung der HPLC mit Standardbedingungen für analytischen Nachweis lässt sich in dem erfindungsgemäß eingesetzten Johanniskrautextrakt kein Hypericin nachweisen.

Es wurde im Rahmen der vorliegenden Erfindung überraschenderweise gefunden, dass die Kombination der beiden Extrakte einen synergistischen Effekt zeigt, der über eine bloße Addition der Wirksamkeit der einzelnen Extrakte deutlich hinausgeht.

Hyperforin ist eine an sich instabile Substanz. Überraschenderweise wurde gefunden, dass der erfindungsgemäß eingesetzte Extrakt aus Usnea barbata zu einer deutlichen Stabilisierung von Hyperforin beiträgt.

Die Kombination des Hypericum-Extraktes mit dem Usnea-Extrakt steigert die antiinflammatorische Wirkung der Einzelkomponenten um ca. das Zehnfache. Damit stellt die Kombination eines auf Hyperforin eingestellten Hypericum-Extraktes und eines auf Usninsäure eingestellten Usnea-Extraktes eine nicht zu erwartende pharmazeutische Verbesserung dar.

Gegenstand der vorliegenden Erfindung sind pharmazeutische Zusammensetzungen, die dadurch gekennzeichnet sind, dass sie 0,001 - 20 Gew.-% eines CO₂-Extraktes aus Usnea barbata und 0,01-80 Gew.-%, bevorzugt 0,01 bis 20 Gew.-%, eines CO₂-Extraktes aus Hypericum perforatum enthalten.

Eine erfindungsgemäß bevorzugte pharmazeutische Zusammensetzung enthält 0,11 - 10 Gew.-% des Extraktes aus Usnea barbata und 10 - 60 Gew.-% des Extraktes aus Hypericum perforatum. Besonders bevorzugt wird kombiniert 6 Gew.-% des Extraktes aus Usnea barbata und 40 Gew.-% des Extraktes aus Hypericum perforatum.

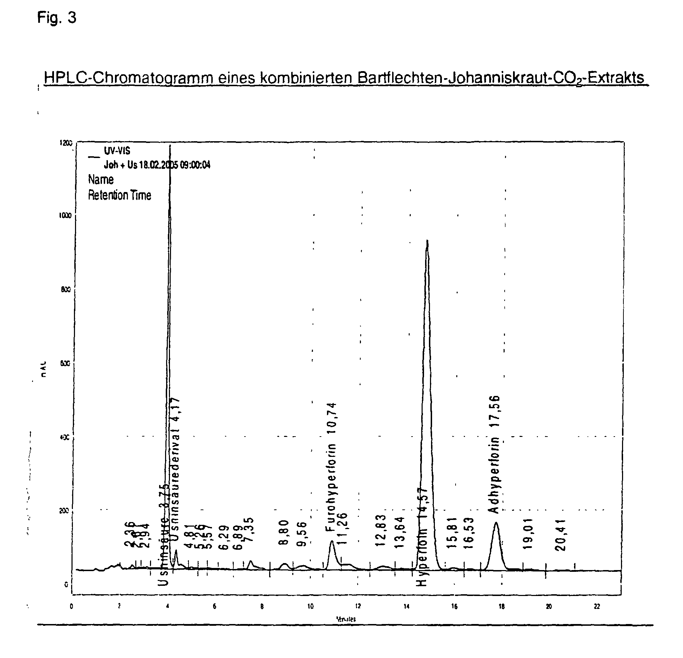

Eine besonders bevorzugte Herstellungsform ist die gemeinsame Homogenisierung von 6 Gew.-% des Extraktes aus Usnea barbata und 40 Gew.-% des Extraktes aus Johanniskraut in einer Kolloidmühle, die folgende Vorteile aufweist: Der nachgewiesenermaßen stabilisierende Effekt der Usninsäure auf den Hypericumextrakt wird sinnvollerweise zu einem möglichst frühen Herstellungszeitpunkt genutzt. Hierzu wird die angereicherte Usninsäure des Bartflechtenextrakts, ein schwerlösliches Pulver, durch Einarbeitung in die Formulierung unter Zusatz einer Pflanzenöl- und Emulgatorenkomponente aufgeschlossen. In der Kolloidmühle werden unter schonenden Bedingungen Usninsäurepartikel von kleiner 5 µm (Micron) erreicht. Im Homogenisat werden analytisch genau die Wirkstoffmengen an Hyperforin und Usninsäure gemessen, die den gemischten Einzelkomponenten entsprechen, d.h. bei der Herstellung des Usnea-Hypericum-Konzentrats ist keine Abbaureaktion zu beobachten. Mit dem Homogenisat erreicht man eine beschleunigte Auflösungskinetik und eine gleichmäßigere Verteilung der Wirkstoffe im Endprodukt. Auf diese Weise wird ein Konzentrat erhalten, das eine ideale Vorformulierung für die Verarbeitung in erfindungsgemäße Zubereitungen zur topischen Behandlung darstellt. Es schränkt die Endformulierung in keiner Weise ein und kann sowohl in hydrophilen als auch in lipophilen Endprodukten verwendet werden. Ein HPLC-Chromatogramm eines derartigen Extraktes ist in Fig. 3 dargestellt.

Das erfindungsgemässe Usnea-Hypericum-Konzentrat wird vorteilhaft in einer Konzentration von 0,01% bis 10% in topischen Grundlagen eingesetzt. Bevorzugt ist eine Konzentration von 0,5% bis 5%, am bevorzugtesten die Konzentration von etwa 2 %. Das entspricht einer Hyperforinkonzentration von etwa 0,3 % und einer Usninsäurekonzentration von etwa 0,1 % im Endprodukt. Diese Gew.-%-Angaben beziehen sich auf das fertige Präparat.

Das Usnea-Hypericum-Konzentrat stellt insbesondere in einer wässrigen Gelgrundlage ein besonders geeignetes Präparat zur Behandlung von Akne und unreiner Haut dar. Ein weiterer Anwendungsbereich des Usnea-Hypericum-Konzentrats ist die Verhinderung der UV-induzierten Entzündung und Hautalterung.

Das Usnea-Hypericum-Konzentrat kann vorteilhaft in andere fettfreie Gelgrundlagen, die dem Fachmann allgemein bekannt sind, eingearbeitet werden. Wegen der Lipophilie der Komponenten ist aber auch die Einarbeitung der Komponenten in Lipidphasen möglich, z.B. in Form von Lipogelen, Cremes, Lotionen und Salben.

Das Usnea-Hypericum-Konzentrat eignet sich nicht nur zur Behandlung mikrobieller Hauterkrankungen, wie Akne und unreine Haut. Wegen der davon unabhängigen ausgeprägten antientzündlichen Wirkung ist die Kombination der Extrakte auch hervorragend geeignet zur Behandlung entzündlicher Hauterkrankungen wie Ekzeme, Lichen ruber und Psoriasis, insbesondere wenn das Gesicht und der behaarte Kopf betroffen sind.

Im übrigen enthält die pharmazeutische Zusammensetzung neben dem Usnea-Hypericum-Konzentrat Zusatzstoffe, die in Abhängigkeit von der gewählten pharmazeutischen Formulierung zugegeben werden. Neben den Grundstoffen für Gels, Salben, Lotionen, Tinkturen etc. können die pharmazeutischen Zusammensetzungen gegebenenfalls Konservierungsstoffe, Antioxidationsmittel, Geruchsstoffe, Färbemittel und ähnliches enthalten. Es ist selbstverständlich, daß sich sämtliche einzelnen Komponenten auf 100 Gew.-% des fertigen Präparats aufaddieren müssen.

In bevorzugter Ausführungsform werden die erfindungsgemäßen pharmazeutischen Zusammensetzungen als topische Formulierungsformen zur Verfügung gestellt. Topische Darreichungsformen werden vor allem bei der lokalen Behandlung von Erkrankungen der Haut und seltener bei systemischen Erkrankungen angewendet.

Dem Pharmazeuten ist eine Vielzahl von topischen Darreichungsformen bekannt. Gängige Ausführungsformen einer topischen Formulierung für dermatologische Erkrankungen sind Salben. Hierbei wird der pharmakologische Wirkstoff in einer halbfesten Salbengrundlage gelöst oder suspendiert. Üblicherweise handelt es sich hierbei um hydrophobe, streichfähige Salben, die die Verdunstung von Wasser aus der Haut verhindern. Gerade bei der Behandlung von Akne sind aber Salben nicht bevorzugt, weil die Salbengrundlage das Auftreten von Hautunreinheiten begünstigen kann. Bei anderen Erkrankungen aber, beispielsweise Schuppenflechte, kann es durchaus vorteilhaft sein, die erfindungsgemäße pharmazeutische Zusammensetzung in eine Salbengrundlage einzuarbeiten. Die Salbengrundlage wird häufig durch flüssige, halbfeste und sogar feste Kohlenwasserstoffe gebildet, die üblicherweise aus Erdöl gewonnen werden. Es können aber auch natürliche Wachse (beispielsweise Bienenwachs oder Jojobaöl) oder synthetische Wachse wie Cetylesterwachse eingesetzt werden. Auch organische Öle, insbesondere Olivenöl oder Baumwollsamenöl können in die Salben eingearbeitet werden. Durch die Mischung der einzelnen Bestandteile kann die Konsistenz der fertigen Salbe beeinflußt werden.

In einer weiteren Ausführungsform kann das erfindungsgemäße Usnea-Hypericum-Konzentrat in Cremes eingearbeitet werden. Hierbei handelt es sich um halbfeste Emulsionen, die flüssiger sind als Salben und leichter auf der Haut verteilbar sind. Die Grundbestandteile der Cremes können kleinere Mengen an Wasser aufnehmen und zu Emulsionen verarbeitet werden. Derartige Cremes sind in der Regel wasserabwaschbar und verstopfen die Poren der Haut nicht. Sie sind nicht fettig und ergeben beim Auftragen auf die Haut ein akzeptables Erscheinungsbild. Üblicherweise bestehen Cremes aus zwei Phasen, nämlich einer ölartigen internen Phase, die bevorzugt aus Kohlenwasserstoffen und hochmolekularen Alkoholen oder Fettsäuren besteht. Weiterhin enthalten die Cremes eine wäßrige Phase, die häufig Konservierungsstoffe, Feuchhaltemittel und Puffermittel enthält. Schließlich weisen die Cremes üblicherweise einen oder mehrere Emulgatoren auf. Häufig werden als Emulgatoren anionische, oberflächenaktive Mittel wie Natriumlaurylsulfat oder Triethanolaminstearat sowie nichtionische Tenside wie beispielsweise Ethylenoxidderivate verwendet. Kationische Tenside wie quaternäre Ammoniumsalze werden weniger bevorzugt, da sie häufig zu Hautirritationen führen können.

Um die pharmazeutischen Wirkstoffe besser lösen zu können, können die Cremes flüssige und wachsartige Komponenten wie beispielsweise Polyethylenglykol enthalten.

In einer bevorzugten Ausführungsform werden die erfindungsgemäßen pharmazeutischen Zusammensetzungen in wäßrige Gele eingearbeitet. Bei den wäßrigen Gelen wird eine flüssige, wässerige interne Phase in einer dreidimensionalen Matrix immobilisiert. Das System wird durch kolloidale Dispersionen von kleinen anorganischen oder großen organischen Molekülen in einem wäßrigen Medium bereitgestellt. Als gelbildende Stoffe werden bevorzugt nichtionische oder anionische Cellulosederivate wie beispielsweise Natriumcarboxymethylcellulose, saure Carboxyvinylpolymere oder andere hydrophile Kolloide wie Magnesiumaluminiumsilikat, Natriumalginat oder Tragant verwendet.

Bevorzugt kann das erfindungsgemäße Usnea-Hypericum-Konzentrat auch in Lotionen oder Lösungen eingesetzt werden, wobei Propylenglykol bevorzugt als Emulgator verwendet werden kann. Durch Verwendung von 5-10% Propylenglykol gelingt es, den lipophilen Extrakt in das Gel zu emulgieren.

In einer anderen Ausführungsform kann das erfindungsgemäße Usnea-Hypericum-Konzentrat auch als Puder Einsatz finden. Gerade bei der Anwendung auf fetter Haut kann es vorteilhaft sein, die pharmazeutische Zusammensetzung als Puder zu formulieren, wobei derartige Puder häufig auf anorganischer Grundlage hergestellt werden. Als Pudergrundlagen werden bevorzugt pulverige, saugfähige und gut deckende, an der Haut haftende ungiftige Stoffe verwendet. Bevorzugt eingesetzt wird Siliciumdioxid, gefällte Kreide, Magnesiumcarbonat, Zinkoxid sowie Talcum und Mischungen dieser Grundstoffe. Bei der pharmazeutischen Formulierung ist darauf zu achten, dass das erfindungsgemäße Usnea-Hypericum-Konzentrat in einer Form vorliegen muss, in der es von den Pudergrundlagen aufgesaugt bzw. adsorbiert werden können. Hierfür kann beispielsweise ein Teil des Usnea-Hypericum-Konzentrates in 9 Teilen 97%iges Ethanol aufgelöst und auf das Siliciumdioxid aufgesprüht werden.

Schließlich kann das erfindungsgemäße Usnea-Hypericum-Konzentrat auch in Form eines wäßrigen Gels bereitgestellt werden, das durch Zugabe geeigneter Stoffe eine feste Struktur erhält, so dass bevorzugt transparente Stifte bereitgestellt werden, die die pharmazeutische Zusammensetzung enthalten und zum Abdecken von speziellen Hautunreinheiten eingesetzt werden können. Derartige Stifte enthalten bevorzugt mehrwertige aliphatische Alkohole mit bevorzugt 2 - 6 Kohlenstoffatomen wie Ethylenglykol, Propylenglykol, Trimethylenglykol, Glycerin und Mischungen dieser Verbindungen.

Zur Versteifung des wäßrigen Gels können auch gesättigte oder ungesättigte höhere Fettsäuren mit bevorzugt 14 bis 22 Kohlenstoffatomen eingesetzt werden. Beispiele hierfür sind Myristinsäure, Palmitinsäure, Stearinsäure, Oleinsäure, Linolensäure oder Mischungen dieser Fettsäuren.

Bevorzugt bei den galenischen Formulierungen ist Einarbeitung des erfindungsgemäßen Usnea-Hypericum-Konzentrates in eine Nanoemulsion. Diese Nanoemulsion lässt sich vorteilhaft in eine wässrige Gelgrundlage einarbeiten.

Eine bevorzugte Gelgrundlage enthält Carbomer 50.0000, 2-Propanol, Propylenglykol und Wasser (siehe Rezepturbeispiel 1). Das erfindungsgemäße Usnea-Hypericum-Konzentrat kann auch vorteilhaft eingearbeitet werden in andere wässrige Zubereitungsformen wie Lotionen, Sprays, Gesichtswässer und Kopfhautemulsionen. Eine besonders bevorzugte wässrige Lösung enthält den kombinierten Extrakt in einer Essenz mit Octyldodecanol, 2-Propanol, Propylenglykol und Wasser.

Für bestimmte Formulierungen kann es vorteilhaft sein, Zinksulfat-Heptahydrat in einer Konzentration von 0,5-1,5 %, bevorzugt 1,0 % zuzusetzen.

Über die Einarbeitung des erfindungsgemäßen Usnea-Hypericum-Konzentrates in wässrige Trägersysteme hinaus eignet es sich also auch zur Einarbeitung in Cremes, Waschlotionen, Lipogele und Salben, deren prinzipielle Zusammensetzungen dem Fachmann allgemein bekannt sind.

Gut geeignet ist das erfindungsgemässe Usnea-Hypericum-Konzentrat auch zur innerlichen Anwendung in Form von Kapseln. Hierzu werden das Konzentrat in geeigneter Konzentration in ein hochwertiges Pflanzenöl eingearbeitet und unter Licht- und Sauerstoffschutz in opake Hartgelatinekapseln eingearbeitet. Ebenso geeignet ist die Einarbeitung des Konzentrates in Form einer Mikroemulsion. Die Darreichung des Konzentrates in Kapseln eignet sich zur Behandlung von Hauterkrankungen, die durch mikrobielle Infektion und Entzündung gekennzeichnet. Die Kapseln eignen sich insbesondere zur inneren Intensivbehandlung besonders hartnäckiger Erkrankungen des seborrhoischen Formenkreises, zum Beispiel Akne und Rosacea.

Das verwendete Usnea-Hypericum-Konzentrat wird nicht anderweitig als Antibiotikum eingesetzt und ist frei von Schadstoffen und Konservierungsmitteln. Aufgrund ihrer Lipophilie penetrieren die Wirkstoffe besonders gut in die Talgdrüsen, wo Sie Ihre antimikrobielle Wirkung entfalten. Gegebenenfalls können die pharmazeutischen Zubereitungen aber noch solche Stoffe enthalten, die die schnelle Aufnahme der Wirkstoffe erhöhen.

Mit den pharmazeutischen Zusammensetzungen der vorliegenden Erfindung wird ein Produkt auf natürlicher Basis bereitgestellt, das gegen die beim seborrhoischen Formenkreis relevanten Keime hoch wirksam ist und davon unabhängig zusätzlich entzündungshemmende Wirkung aufweist. Die entzündungshemmende Wirkung wurde durch den Proliferationstest mit Lymphozyten, durch die Hemmung der UV-induzierten Prostaglandinsynthese und im Modell des UV-Erythemtests nachgewiesen.

Präparate mit Usnea-Hypericum-Konzentrat liefern somit eine Basis zur Behandlung von Akne und ähnlichen Hauterkrankungen auf natürlicher Grundlage. Im verwendeten Usnea-Hypericum-Konzentrat kommt gleichzeitig sowohl eine ausgezeichnete Wirkung gegen Aknebakterien, als auch gegen Pityrosporon-Hefepilze zur Geltung. Der Extrakt wirkt auch enzündungshemmend, ohne die Haut zu reizen. Ausserdem können Präparate mit Usnea-Hypericum-Konzentrat der UV-induzierten Entzündung und der beschleunigten Hautalterung entgegenwirken.

Besonders vorteilhaft bei Anwendung im Gesicht ist die Kombination des Usnea-Hypericum-Konzentrates mit einem Lichtschutzfaktor. Geeignete Lichtschutzfaktoren können organischchemische und/oder mineralische UV-Filter sein und sind dem Fachmann hinlänglich bekannt.

In einer weiteren bevorzugten Ausführung der Erfindung wird noch ein CO₂-Extrakt aus der Ratanhia-Wurzel (Krameria lappacea) als Lichtschutzfaktor zugesetzt. Der CO₂-Ratanhia-Extrakt enthält keine polymeren Catechin-Gerbstoffe, die bei empfindlicher Haut eher unerwünscht sind, sondern lipophile Phenole vom Neolignan und Norneolignantyp. Der Ratanhia-Extakt ist somit ein gut verträglicher, hochwertiger UVA2 und UVB Filter mit hoher Fotostabilität, dessen Filterwirkung bei Wellenlängen kleiner 340 Nanometer liegt. Die Durchlässigkeit der längerwelligen UVA1 Strahlung im Bereich von 340-400 Nanometer ist bei vielen Indikationen durchaus wünschenswert. Der Ratanhia-Extrakt unterstützt zusätzlich die antimikrobielle und antiinflammatorische Wirkung des beanspruchten Usnea-Hypericum-Konzentrats und kann durch UV-Protektion in hydrophilen Endformulierungen einen zusätzlichen Beitrag zur Hyperforinstabilität leisten. Der CO₂-Ratanhia-Extrakt wird analog wie die anderen hier beschriebenen CO₂-Extrakte hergestellt und enthält wenigstens 60, bevorzugt wenigstens 80 Gew.-% an aktiven Bestandteilen.
Figur 1 zeigt das HPLC-Chromatogramm eines angereicherten CO₂-Extrakts aus Bartflechten mit einem Gehalt von etwa 96,8 Gew.-% an Gesamt-Usninsäuren.
Figur 2 zeigt das HPLC-Chromatogramm eines Johanniskraut-CO₂-Extrakts mit einem Gehalt von etwa 40 % an Gesamt-Hyperforinen.
Figur 3 zeigt ein HPLC-Chromatogramm eines CO₂-Extrakts, bei dem Johanniskraut und Bartflechte zusammen homogenisiert wurden.
Figur 4 zeigt die antiproliferative Wirkung von CO₂-Extrakten wobei einmal ein Hypericum-CO₂-Extrakt, einmal ein Usnea-CO₂-Extrak und einmal ein CO₂-Extrakt eingesetzt wurde, der aus Hypericum und Usnea isoliert wurde. Die Werte wurden im ATP-Assay (ViaLight) anhand der Luminiszenz gemessen.
Figur 5 zeigt den entzündungshemmenden Effekt eines Usnea-Extrakts, der in einer Konzentration von 5 % in einem wässrigen Gel auf die Haut der Probanden aufgebracht wurde. Eine durch UVB-Strahlen hervorgerufene Entzündung kann durch die erfindungsgemäße pharmazeutische Zusammensetzung behandelt werden. Die erfindungsgemäßen pharmazeutischen Zusammensetzungen eignen sich daher auch zur Behandlung von durch übermäßige Sonnenbestrahlung hervorgerufene Entzündungen (Sonnenbrand).
Figur 6 zeigt die dosisabhängige Hemmung der UV-induzierten Prostaglandin-Synthese durch Usnea-Extrakt.
Figur 7 zeigte die Hemmung der UV-induzierten Aufregulation der Matrix-Metalloproteinase-1 (MMP-1) durch Usnea-Extrakt und die Potenzierung der Wirkung durch Kombination von Usnea-Extrakt und Hypericum-Extrakt.

Die nachfolgenden Beispiele sollen die Erfindung näher erläutern.

### Herstellungsbeispiel 1: wässriges Gel mit Propylenglykol

| | | |
|---|---|---|
| a) | 3 % Hypericum-Extrakt | |
| | 2 % Usnea barbata-Extrakt | |
| | Gelgrundlage ad 100,0 | |

| | Gelgrundlage | |
|---|---|---|
| | Carbomer 50.000 | 0,5 |
| | 2-Propanol | 5,0 |
| | Propylenglykol | 20,0 |
| | Natriumhydroxid | 0,12 |
| | Gereinigtes Wasser | ad 100,0 |
| | | |
| b) | 1 % Usnea-Hypericum-Konzentrat | |
| | Gelgrundlage ad 100,0 | |

| | Gelgrundlage | |
|---|---|---|
| | Carbomer 50.000 | 0,5 |
| | 2-Propanol | 5,0 |
| | Propylenglykol | 20,0 |
| | Natriumhydroxid | 0,12 |
| | Gereinigtes Wasser | ad 100,0 |

### Herstellungsbeispiel 2: Gesichtswasser

| | | |
|---|---|---|
| a) | 3 % Hypericum-Extrakt | |
| | 2 % Usnea barbata-Extrakt | |
| | 1 % Zinksulfat-Heptahydrat | |
| | | |
| | Alkoholische Essenz ad 100,0 | |
| | | |

| | Alkoholische Essenz: | |
|---|---|---|
| | Octyldodecanol | 18,0 |
| | 2-Propanol | 58,0 |
| | Propylenglykol | 10,0 |
| | Gereinigtes Wasser | ad 100,0 |
| | | |
| | Eingesetzte Extrakte: | |
| | Johanniskrautextrakt: "St. John's wort CO₂ to extract", Firma FLAVEX Usnea-Extrakt: "Usnea lichen CO₂ to extract water soluble", Firma FLAVEX | |
| | | |
| b) | 2 % Usnea-Hypericum-Konzentrat | |
| | 1 % Zinksulfat-Heptahydrat | |
| | Alkoholische Essenz ad 100,0 | |
| | Octyldodecanol | 18,0 |
| | 2-Propanol | 58,0 |
| | Propylenglykol | 10,0 |
| | Gereinigtes Wasser | ad 100,0 |

### Beispiel 1. Antimikrobielle Wirkung des Bartflechten CO₂-Extraktes:

Der Bartflechten CO₂-Extrak (Usnea barbata L.) wurde in den Konzentrationen 1:10, 1:50, 1:100, 1:500 und 1:1000 im Agardilutionstest bei einer Reihe von aeroben und anaeroben Bakterien geprüft. Die Reinsubstanz Usninsäure wurde in Konzentrationen von 128/ 64/ 32/ 16/ 8/ 4/ 2/ 1/ 0.5/ 0.25 und 0.125 µg/mL getestet. Die MHK (Minimale Hemmkonzentration) ist definiert als > 90 % Hemmung des Bakterienwachstums nach 24h. Die MBK (Minimale Bakterizide Konzentration) ist definiert als > 90 % Hemmung des Wachstums bei 48h. Es zeigte sich überraschend, dass Propionibacterium acnes hochempfindlich auf den Usneaextrakt und auf Usninsäure reagiert. Dies ist in Tabelle 1 dargestellt:

**Tabelle 1: Wirksamkeit von Usnea-Extrakt und Usninsäure auf Propionibacterium acnes**

| **Keimname** | **Bartflechte-Extrakt MHK** | **Bartflechte-Extrakt MBK** | **Usninsäure MHK** | **Usninsäure MBK** |
|---|---|---|---|---|
| Propionibacterium acnes | 1:2500 | 1:1000 | 1 µg/mL | 4 µg/mL |

Ausserdem wurde die Wirksamkeit gegen Pityrosporon von 1 % Usneaextrakt in Olivenöl im Vergleich zu 1 % Amphotericin untersucht. Hierzu wurden Schüppchen von Patienten mit seborrhoischem Ekzem auf einien Sabouraud-Agar aufgebracht und mit dem Öl überschichtet. Nach 4 Tagen Inkubation im Brutschrank wurde die Anzahl der koloniebildenden Einheiten ausgezählt. Das Wachstum der Pityrosporon-Hefepilze wurde vollständig durch den Usneaextrakt und Amphotericin, nicht aber durch Olivenöl alleine gehemmt. Dies ist in Tabelle 2 dargestellt:

**Tabelle 2: Wirksamkeit von Usnea-Extrakt auf Pityrosporon-Hefepilze im Öl-Submersionstext**

| **Keimname** | **1% Bartflechten-Extrakt in Olivenöl** | **1% Amphotericin in Olivenöl** | **Olivenöl ohne Zusatz** |
|---|---|---|---|
| Pityrosporon ovale | 0 KBE | 1 KBE | 29 KBE |

### Beispiel 2. Potenzierung der antiproliferativen Wirkung durch Kombination von Hypericumextrakt und Usneaextrakt:

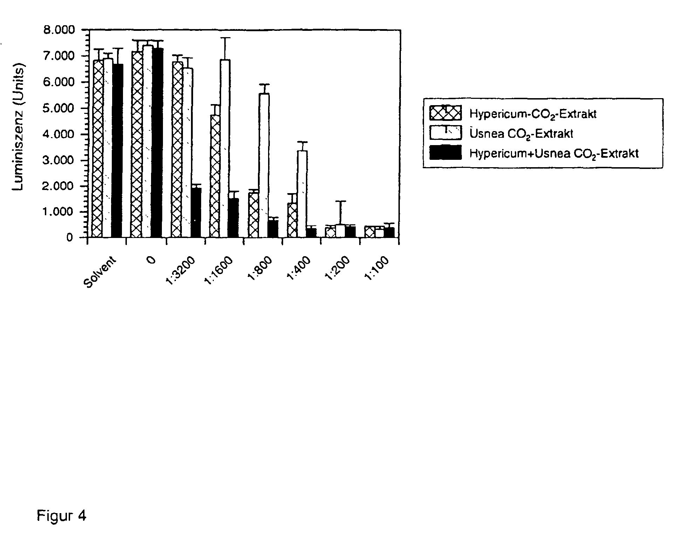

Eine Hemmung der Proliferation aktivierter Lymphozyten kann Ausdruck einer entzündungshemmenden Wirkung von Substanzen sein. Deshalb wurde die proliferationshemmende Wirkung von Usneaextrakt untersucht. Ausserdem wurde untersucht, ob Usneaextrakt die bekannte proliferationshemmende Wirkung eines Johanniskraut (Hypericum)-CO₂-Extraktes verstärken kann. Hierzu wurden Lymphozyten des peripheren Blutes (PBMC) über Ficoll-Dichtegradienten isoliert und in einer Zellzahl von 100.000/mL in Mikrotiterplatten ausgesät. Danach wurden die Zellen mit 1 µg/mL PHA (Phytohämagglutinin) stimuliert und mit oder ohne Zusatz von Pflanzenextrakten bzw. Lösungsmittelkontrolle für 24 h inkubiert. Folgende Stammlösungen wurden hergestellt: 25 % Usneaextrakt in 70 % EtOH, 25 % Hypericumextrakt in EtOH, 12,5 % Usneaextrakt+12,5 % Hypericumextrakt in EtOH. Alle Stammlösungen wurden in einer Verdünnungsreihe von 1:100 bis 1:3200 eingesetzt. Danach wurde die Zellproliferation durch Messung des ATP-Gehaltes (ViaLight) bestimmt. Die halbe Hemmkonzentration (IC50) von Usneaextrakt tritt bereits bei einer Verdünnung von 1:400 auf. Es zeigte sich überraschend, dass der Zusatz von Usneaextrakt zum Hypericumextrakt nicht nur additiv wirkt, sondern dessen antiproliferative Wirkung potenziert (Figur 4).

### Beispiel 3. Entzündungshemmende Wirkung im UV-Erythemtest:

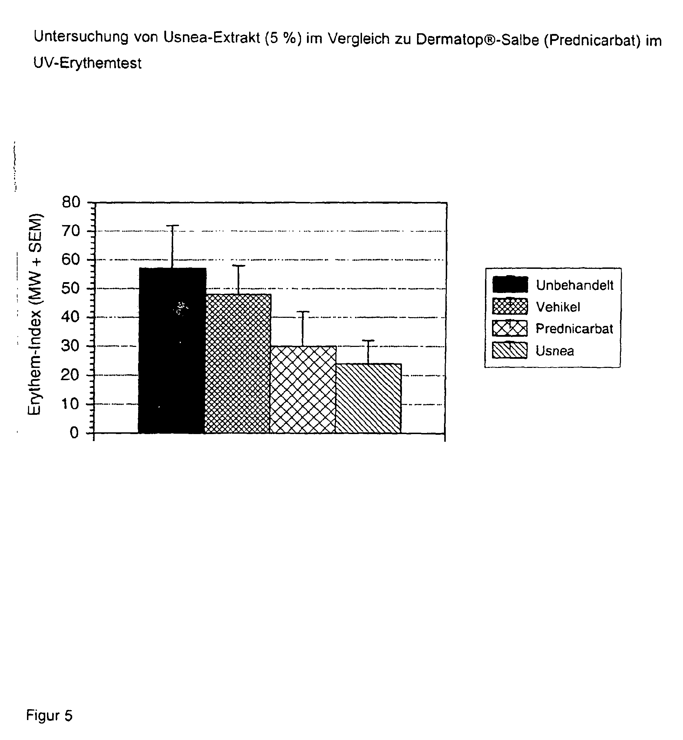

Bei 7 gesunden Probanden wurde eine Lichttreppe mit UVB durchgeführt. Anschliessend wurden Testfelder auf dem Rücken mit der 1,5 fachen minimalen Erythemdosis (MED) bestrahlt. Die Testsubstanzen wurde in Finn-Chambers für 24 Stunden aufgebracht. Das UV-induzierte Erythem wurde vor Bestrahlung und nach Einwirken der Substanzen photometrisch bestimmt. Es zeigte sich, dass 5 % Usneaextrakt so gut wie das Cortisonpräparat Prednicarbat (Dermatop®) die UV-induzierte Entzündung hemmen kann (Figur 5).

### Beispiel 4. Stabilisierung von Hyperforin durch Zusatz von Usnea-Extrakt:

Es wurde überraschend gefunden, dass die Kombination eines auf 10 % Hyperforin standardisierten Johanniskrautextraktes mit einem auf 4 % Usninsäure standardisierten Usnea-Extrakt bei Einarbeitung in ein wässriges Gel mit Propylenglykol zu einer Zunahme der Stabilität von Hyperforin um 100 % führt (Tabelle 3).

Tabelle 3 zeigt die stabilisierende Wirkung eines Usnea-CO₂-Extrakts auf einen CO₂-Extrakt aus Johanniskraut. In Tabelle 3 ist deutlich zu sehen, dass der Anteil von Hyperforin fast doppelt so hoch ist, wenn Usnea-CO₂-Extrak zugesetzt wurde. Diese zeitliche Stabilisierung ist für pharmazeutische und kosmetische Produkte sehr wichtig, da diese möglichst lange ohne Abbau der Wirkstoffe haltbar sein sollen.

**Tabelle 3: Stabilisierung von Hyperforin durch Zusatz von Usnea-Extrakt:**

| | kurz nach Herstellung | | nach 8 Wochen | |
|---|---|---|---|---|
| | | | | |

| | mit Usnea | ohne Usnea | mit Usnea | ohne Usnea |
|---|---|---|---|---|
| **Hyperforine** | **43%** | **42%** | **36%** | **18%** |
| andere Komponenten sowie Hyperforinabbauprodukte | 57% | 58% | 64% | 82% |

### Beispiel 5. Stabilisierung von Hyperforin im Lichtbelastungstest durch die Kombination von Hypericum-Extrakt und Usnea-Extrakt

Das erfindungsgemäß bevorzugte, über eine Kolloidmühle vorformulierte Usnea-Hypericum-Konzentrat wurde in der bevorzugten Konzentration von 2 % in MCT-ÖI gelöst, um ein anwendungsfertiges Endprodukt zu simulieren. Der Hyperforingehalt der Endformulierung wurde mit 0,305 % ermittelt. Im Vergleich wurde die identisch hergestellte Vorformulierung ohne Usnea-Extrakt nur mit Hypericum ebenfalls 2 %-ig in MCT-Öl gelöst, wobei ein Hyperforingehalt von 0,313 % in der Endformulierung gemessen wurde. Von beiden dünnflüssigen Ölformulierungen wurden jeweils 5 g auf eine Glasplatte gegossen, wo sie einen dünnen Film von ca. 1 mm Schichtdicke ausbilden. Beide Glasplatten wurden im Freien dem Tageslicht ausgesetzt, wobei der dünne Ölfilm eine große Angriffsfläche für Licht und Sauerstoff bildet. Von dem Ölfilm auf beiden Platten wurde in bestimmten Zeitabständen Proben entnommen und der Hyperforingehalt mittels HPLC gemessen. Auf diese Weise kann der Hyperforinabbau in beiden Endformulierungen verfolgt werden. Am Ende der Meßreihen wurde in der Formulierung ohne Usnea, nur mit Hypericum ein Gehalt von 0,057 % Hyperforin gefunden, während der Hyperforingehalt in der Hypericum-Usnea-Zubereitung nach der gleichen Zeitspanne mit 0,117 % mehr als doppelt so hoch ausfiel. Damit ist nochmals die bessere Stabilität des Hyperforins in dem Hypericum-Usnea-Komplex auch in verdünnter Anwendungsformulierung belegt.

### Beispiel 6. Hemmung der UV-induzierten Prostaglandin E₂ (PGE₂)-Produktion durch Usnea-Extrakt:

HaCaT Keratinozyten (100.000/ mL) wurden in Petrischalen mit Zellkulturmedium (RPMI mit 10% FBS) bis zur Konfluenz kultiviert. Anschliessend wurde das Medium durch Phosphatpuffer ersetzt und die Cyclooxygenase durch Zusatz von Arachidonsäure (AA) gesättigt. Ein Ansatz wurde im Dunkeln belassen, ein Ansatz wurde mit 30 mJ/cm2 UVB bestrahlt. Beide Ansätze wurden nach Zugabe von Medium und Zusatz von Usneaextrakt in unterschiedlichen Konzentrationen für 24h inkubiert. Dann wurden die Überstände abgenommen und die Produktion von PGE2 mit einem PGE₂ ELISA (R&D Systems) gemessen.

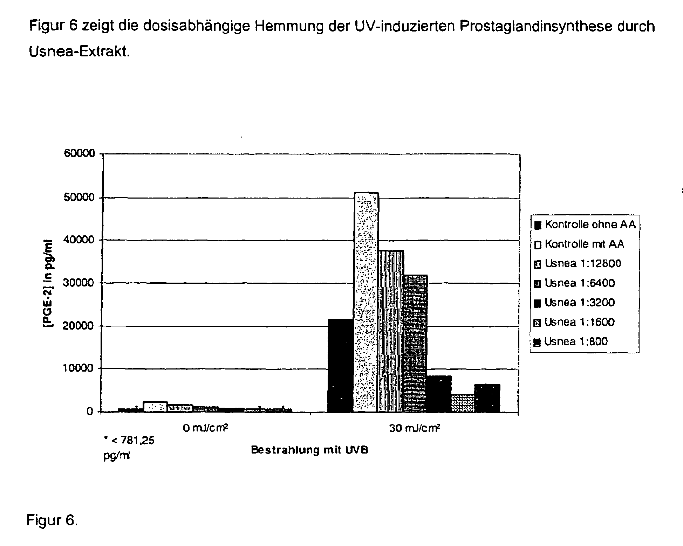

Figur 6 zeigt die dosisabhängige Hemmung der UV-induzierten Prostaglandinsynthese durch Usnea-Extrakt.

### Beispiel 7. Hemmung der UV-induzierten Aufregulation der Matrix-Metalloproteinase (MMP-1) durch Usnea-Extrakt und die Kombination von Usnea-Extrakt und Hypericum-Extrakt:

Primäre humane Fibroblasten wurden durch enzymatische Verdauung aus humaner Haut isoliert und in Zellkulturmedium (DMEM mit 10% FBS) über mehrere Passagen in Petrischalen kultiviert. Dann wurden 100.000 Zellen/ mL in Petrischalen ausgesät und nach Adhärenz mit Medium, Usnea-Extrakt und Hypericum Extrakt inkubiert. In einem Ansatz wurde das Medium durch Phosphatpuffer ersetzt und die Zellen wurden mit 60 J/cm2 UVA-1 bestrahlt. Nach Zugabe von Medium wurde ein Teil der bestrahlten Zellen mit Usnea-Extrakt oder der Kombination von Usnea-Extrakt und Hypericum Extrakt in einer Konzentration von jeweils 0,1% v/v für 24h inkubiert. Anschliessend wurden die Zellen auf Eis lysiert und die Aktivität der MMP-1 durch Messung mit einem MMP-1 Enzym-Aktivitätsassay (R&D Systems) bestimmt.

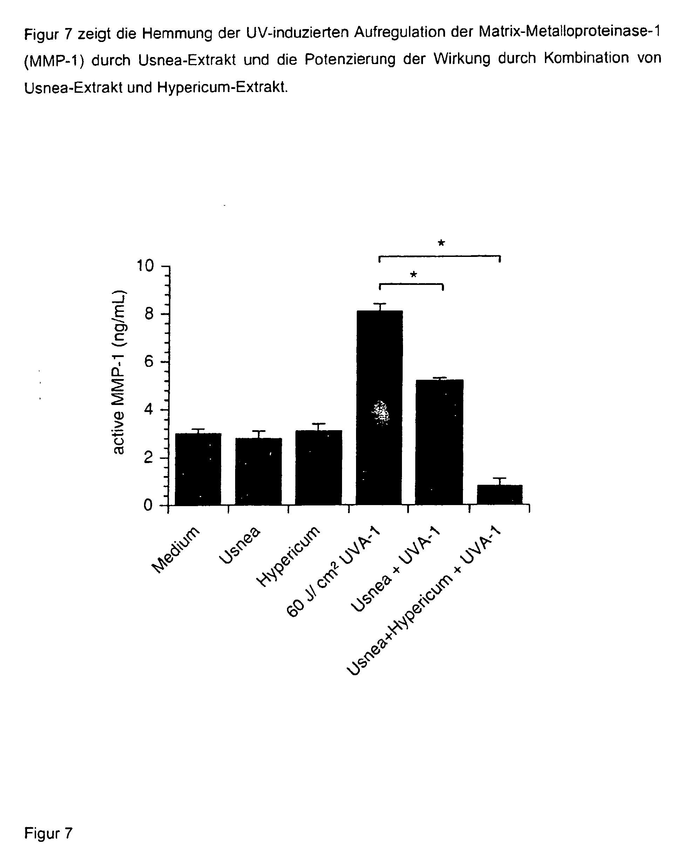

Figur 7 zeigt die Hemmung der UV-induzierten Aufregulation der Matrix-Metalloproteinase-1 (MMP-1) durch Usnea-Extrakt und die Potenzierung der Wirkung durch Kombination von Usnea-Extrakt und Hypericum-Extrakt.

Darüber hinaus zeigt Figur 5 den entzündungshemmenden Effekt eines Usnea-CO₂-Extrakts, der in einer Konzentration von 5 % in einem wässrigen Gel auf die Haut der Probanden aufgebracht wurde. Deshalb kann eine durch UVB-Strahlen hervorgerufene Entzündung durch die erfindungsgemäße pharmazeutische Zusammensetzung behandelt werden. Die erfindungsgemäße pharmazeutische Zusammensetzung eignet sich daher auch zur Behandlung von durch übermäßige Sonnenbestrahlung hervorgerufene Entzündungen (Sonnenbrand).

### Beispiel 8. Anwendung von Usnea-Gel und Usnea/Hypericum-Gel bei Akne vulgaris:

Bei 3 Patienten kam es durch Anwendung eines erfindungsgemässen Gels gemäss Herstellungsbeispiel 1 in zwei Wochen zur Abheilung der Hautveränderungen im Gesicht.

Die Ergebnisse sind in Tabelle 4 dargestellt.

**Tabelle 4**

| | Alter, Geschlecht | vor Behandlung | nach zwei Wochen |
|---|---|---|---|
| Patient 1 | 30 Jahre, männlich | Akne vulgaris | gebessert |
| Patient 2 | 24 Jahre, weiblich | Akne papulopustuolosa | abgeheilt |
| Patient 3 | 56 Jahre, weiblich | Rosacea | abgeheilt |

## Patentansprüche

1. Pharmazeutische Zusammensetzung, **dadurch gekennzeichnet, dass** sie 0,001 - 20 Gew.-% eines CO₂-Extraktes aus Usnea barbata und 0,01 - 80 Gew.-% eines CO₂-Extraktes aus Johanniskraut (Hypericum perforatum) enthält.

2. Pharmazeutische Zusammensetzung nach Anspruch 1, **dadurch gekennzeichnet, dass** sie 0,11 - 10 Gew.-% eines Extraktes aus Usnea barbata und 0,11 - 10 Gew.-% eines Extraktes aus Johanniskraut (Hypericum perforatum) enthält.

3. Pharmazeutische Zusammensetzung nach Anspruch 1, **dadurch gekennzeichnet, dass** sie 0,5 - 5 Gew.-% eines Extraktes aus Usnea barbata und 0,5 - 5 Gew.-% eines Extraktes aus Johanniskraut (Hypericum perforatum) enthält.

4. Pharmazeutische Zusammensetzung nach Anspruch 1, **dadurch gekennzeichnet, dass** der CO₂-Extrakt aus Usnea barbata 3 - 6 Gew.-% Usninsäure enthält.

5. Pharmazeutische Zusammensetzung nach Anspruch 1, **dadurch gekennzeichnet, dass** der CO₂-Extrakt aus Usnea barbata wenigstens 85 Gew.-% Usninsäure enthält.

6. Pharmazeutische Zusammensetzung nach Anspruch 1, **dadurch gekennzeichnet, dass** der CO₂-Extrakt aus Johanniskraut (Hypericum perforatum) 5 - 15 Gew.-% Hyperforin enthält.

7. Pharmazeutische Zusammensetzung nach Anspruch 1, **dadurch gekennzeichnet, dass** der CO₂-Extrakt aus Johanniskraut (Hypericum perforatum) wenigstens 40 Gew.-% Hyperforin enthält.

8. Pharmazeutische Zusammensetzung nach einem der vorhergehenden Ansprüche, **dadurch gekennzeichnet, dass** es sich um ein wässriges Gel handelt.

9. Pharmazeutische Zusammensetzung nach einem der Ansprüche 1 bis 7, **dadurch gekennzeichnet, dass** es sich um eine Lotion handelt.

10. Pharmazeutische Zusammensetzung nach einem der Ansprüche 1 bis 7, **dadurch gekennzeichnet, dass** es sich um eine Salbe handelt.

11. Pharmazeutische Zusammensetzung nach einem der Ansprüche 1 bis 7, **dadurch gekennzeichnet, dass** es sich um ein versteiftes Gel handelt, das in Form eines Stiftes einsetzbar ist.

12. Pharmazeutische Zusammensetzung nach einem der Ansprüche 1 bis 7, **dadurch gekennzeichnet, dass** es sich um einen Puder handelt.

13. Verwendung einer Zusammensetzung nach einem der Ansprüche 1 bis 12 zur Herstellung eines Medikaments zur Behandlung einer Hauterkrankung des seborrhoischen Formenkreises.

14. Verwendung nach Anspruch 13, **dadurch gekennzeichnet, dass** es sich bei der Erkrankung um Akne handelt.

15. Verwendung nach einem der Ansprüche 13 oder 14, **dadurch gekennzeichnet, dass** es sich um eine topisch anwendbare Formulierung handelt.

16. Verwendung einer pharmazeutischen Zusammensetzung nach einem der Ansprüche 1 bis 7 zur Herstellung eines Medikaments zur Behandlung des seborrhoischen Formenkreises, **dadurch gekennzeichnet, dass** es sich um ein oral verabreichbares Medikament handelt.

17. Verwendung einer Zusammensetzung nach einem der Ansprüche 1 bis 12 zur Herstellung eines Medikaments zur Behandlung der strahlengeschädigten Haut.

18. Verwendung nach Anspruch 17, **dadurch gekennzeichnet, dass** die Haut durch Sonnenstrahlung geschädigt wurde.

19. Verwendung einer Zusammensetzung nach einem der Ansprüche 1 bis 12 zur Herstellung eines Medikaments zur Behandlung und Vorbeugung von Alterungserscheinungen der Haut.

## Claims

1. Pharmaceutical composition, **characterized in that** it contains 0.001-20% by weight of a CO₂ extract of old man's beard and 0.01-80% by weight of a CO₂ extract of St. John's Wort (Hypericum perforatum).

2. Pharmaceutical composition according to Claim 1, **characterized in that** it contains 0.11-10% by weight of an extract of old man's beard and 0.11-10% by weight of an extract of St. John's Wort (Hypericum perforatum).

3. Pharmaceutical composition according to Claim 1, **characterized in that** it contains 0.5-5% by weight of an extract of old man's beard and 0.5-5% by weight of an extract of St. John's Wort (Hypericum perforatum).

4. Pharmaceutical composition according to Claim 1, **characterized in that** the CO₂ extract of o!d man's beard contains 3-6% by weight of usnic acid.

5. Pharmaceutical composition according to claim 1, **characterized in that** the CO₂ extract of old man's beard contains at least 85% by weight of usnic acid.

6. Pharmaceutical composition according to Claim 1, **characterized in that** the CO₂ extract of St. John's Wort contains 5-15% by weight of hyperforin.

7. Pharmaceutical composition according to Claim 1, **characterized in that** the CO₂ extract of St. John's Wort contains at least 40% by weight of hyperforin.

8. Pharmaceutical composition according to any of the preceding Claims, **characterized in that** it is an aqueous gel.

9. Pharmaceutical composition according to any of Claims 1 to 7, **characterized in that** it is a lotion.

10. Pharmaceutical composition according to any of Claims 1 to 7, **characterized in that** it is an ointment.

11. Pharmaceutical composition according to any of Claims 1 to 7, **characterized in that** it is a stiffened gel which can be used in the form of a pen.

12. Pharmaceutical composition according to any of Claims 1 to 7, **characterized in that** it is a powder.

13. Use of a composition according to any of Claims 1 to 12 for the preparation of a medicament for the treatment of a skin disease of the seborrhoeal group.

14. Use according to Claim 13, **characterized in that** the disease is acne.

15. Use according to either of Claims 13 and 14, **characterized in that** it is a topically applicable formulation.

16. Use of a pharmaceutical composition according to any of Claims 1 to 7 for the preparation of a medicament for the treatment of the seborrhoeal group, **characterized in that** it is a medicament which can be administered orally.

17. Use of a composition according to any of Claims 1 to 12 for the preparation of a medicament for the treatment of radiation-damaged skin.

18. Use according to Claim 17, **characterized in that** the skin was damaged by sunlight.

19. Use of a composition according to any of Claims 1 to 12 for the preparation of a medicament for the treatment and prevention of ageing phenomena of the skin.

## Revendications

1. Composition pharmaceutique, **caractérisée en ce qu'**elle contient de 0,001 à 20% en poids d'un extrait au CO₂ d'Usnea barbata et de 0,01 à 80% en poids d'un extrait au CO₂ de millepertuis (Hypericum perforatum).

2. Composition pharmaceutique suivant la revendication 1, **caractérisée en ce qu'**elle contient de 0,11 à 10% en poids d'un extrait d'Usnea barbata et de 0,11 à 10% en poids d'un extrait de millepertuis (Hypericum perforatum).

3. Composition pharmaceutique suivant la revendication, **caractérisée en ce qu'**elle contient de 0,5 à 5% en poids d'un extrait d'Usnea barbata et de 0,5 à 5% en poids d'un extrait de millepertuis (Hypericum perforatum).

4. Composition pharmaceutique suivant la revendication 1, **caractérisée en ce que** l'extrait au CO₂ d'Usnea barbata contient de 3 à 6% en poids d'acide usninique.

5. Composition pharmaceutique suivant la revendication 1, **caractérisée en ce que** l'extrait au CO₂ d'Usnea barbata contient au moins 85% en poids d'acide usninique.

6. Composition pharmaceutique suivant la revendication 1, **caractérisée en ce que** l'extrait au CO₂ de millepertuis (Hypericum perforatum) contient de 5 à 15% en poids d'hyperforine.

7. Composition pharmaceutique suivant la revendication 1, **caractérisée en ce que** l'extrait au CO₂ de millepertuis (Hypericum perforatum) contient au moins 40% en poids d'hyperforine.

8. Composition pharmaceutique suivant l'une quelconque des revendications qui précèdent, **caractérisée en ce qu'**il s'agit d'un gel aqueux.

9. Composition pharmaceutique suivant l'une quelconque des revendications 1 à 7, **caractérisée en ce qu'**il s'agit d'une lotion.

10. Composition pharmaceutique suivant l'une quelconque des revendications 1 à 7, **caractérisée en ce qu'**il s'agit d'une pommade.

11. Composition pharmaceutique suivant l'une quelconque des revendications 1 à 7, **caractérisée en ce qu'**il s'agit d'un gel solidifié qui peut être utilisé sous forme d'un bâton.

12. Composition pharmaceutique suivant l'une quelconque des revendications 1 à 7, **caractérisée en ce qu'**il s'agit d'une poudre.

13. Utilisation d'une composition suivant l'une quelconque des revendications 1 à 12 pour la préparation d'un médicament destiné au traitement d'une maladie de peau de type séborrhéique.

14. Utilisation suivant la revendication 13, **caractérisée en ce que** la maladie est de l'acné.

15. Utilisation suivant l'une quelconque des revendications 13 ou 14, **caractérisée en ce qu'**il s'agit d'une formulation utilisable localement.

16. Utilisation d'une composition pharmaceutique suivant l'une quelconque des revendications 1 à 7 pour la préparation d'un médicament pour le traitement d'affections de type séborrhéique, **caractérisée en ce qu'**il s'agit d'un médicament administrable par voie orale.

17. Utilisation d'une composition suivant l'une quelconque des revendications 1 à 12 pour la préparation d'un médicament destiné au traitement d'une peau abîmée par un rayonnement.

18. Utilisation suivant la revendication 17, **caractérisée en ce que** la peau a été abîmée par le rayonnement solaire.

19. Utilisation d'une composition suivant l'une quelconque des revendications 1 à 12 pour la préparation d'un médicament destiné au traitement et à la prévention de symptômes de vieillissement de la peau.
